## Europäisches Patentamt

## European Patent Office

## Office européen des brevets

(19)

(11) Veröffentlichungsnummer: **0 168 726**
**B1**

(12) **EUROPÄISCHE PATENTSCHRIFT**

(45) Veröffentlichungstag der Patentschrift:
27.12.89

(21) Anmeldenummer: 85108349.3

(22) Anmeldetag: 05.07.85

(51) Int. Cl.⁴: **C 04 B 28/26,** C 04 B 30/00

(54) Formkörper aus silikatischem Material, ihre Verwendung und Verfahren zu ihrer Herstellung.

(30) Priorität: 18.07.84  DE 3426389

(43) Veröffentlichungstag der Anmeldung:
22.01.86 Patentblatt 86/4

(45) Bekanntmachung des Hinweises auf die Patenterteilung:
27.12.89 Patentblatt 89/52

(84) Benannte Vertragsstaaten:
BE CH DE FR GB IT LI NL SE

(56) Entgegenhaltungen:
DE-A-2 143 346
DE-A-3 400 132

(73) Patentinhaber: HOECHST AKTIENGESELLSCHAFT,
Postfach 80 03 20, D-6230 Frankfurt am Main 80
(DE)

(72) Erfinder: Rieck, Hans- Peter, Dr., Staufenstrasse
13a, D-6238 Hofheim am Taunus (DE)
Erfinder: Schott, Martin, Dr., Königsteiner Strasse
7, D-6374 Steinbach/Taunus (DE)
Erfinder: Russow, Jürgen, Dr., Am Schieferberg
45, D-6233 Kelkheim (Taunus) (DE)

LIBER, STOCKHOLM 1989

**Beschreibung**

Die vorliegende Erfindung betrifft Formkörper aus Kieselsäuren mit Schichtstruktur oder deren Salzen, ihre Verwendung und Verfahren zu ihrer Herstellung.

Die hier verwendeten Kieselsäuren und ihre Salze unterscheiden sich durch ihre Schichtstruktur von amorphen Kieselsäuren oder Kieselsäuren mit Gerüststruktur, bzw. deren Salzen, wie z. B. Silicalit (US-PS 4 061 724).

Solche Schichtkieselsäuren und deren Alkalisalze sind in der Natur gefunden worden (H.P. Eugster, Science *157*, 1177 - 1180; T.P. Rooney et al., Amer. Mineral., *54*, 1034 - 1043 (1969); G. Maglione und M. Servant, C.R. Acad. Sci., Ser. D., *277*, 1721 - 1724 (1973); J.L. McAtee, Jr. et al., Amer. Mineral., 53 (1968), 2061 - 2069.

Für die dort beschriebenen Mineralien Kenyait und Magadiit werden u.a. von Eugster die Formeln $NaSi_{11}O_{20,5}(OH)_4 \cdot 3H_2O$ und $NaSi_7O_{13}(OH)_3 \cdot 3H_2O$ angegeben. Dabei wird unterschieden zwischen chemisch gebundenem Wasser (OH-Gruppen) und Kristall-Wasser. Diese Unterscheidung ist jedoch nicht mit Sicherheit durchführbar.

Man kann die gleichen Substanzen auch als $Na_2Si_{22}O_{45} \cdot 10H_2O$ und $Na_2Si_{14}O_{29} \cdot 9H_2O$ formulieren.

Eine Reihe von Alkalisalzen und Kieselsäuren mit Schichtstruktur sind auch schon synthetisch erhalten worden. Aus diesen Alkalisilikaten können durch sauren Ionenaustausch die freien Kieselsäuren gewonnen werden. Dabei bleibt die Schichtstruktur der Verbindungen erhalten.

Ein auch technisch gangbares Verfahren zur Herstellung von kristallinen Alkalischichtsilikaten mit Molverhältnissen $Na_2O/SiO_2$ von 1 : 14 bis 1 : 22 ist Gegenstand der deutschen Patentanmeldung P 3 400 132.8 (EP-A-151 295). Nach diesem Verfahren wird ein in Wasser gelöstes oder ein amorphes Alkalisilikat mit einem molaren Verhältnis $M_2O/SiO_2$, wobei M für ein Alkalimetall steht, von 0,24 bis 2,0 mit so viel einer sauren Verbindung versetzt, daß ein molares Verhältnis $M_2O$ (nicht neutralisiert) / $SiO_2$ von 0,05 bis 0,239 erreicht wird, gegebenenfalls durch Verdünnen ein molares Verhältnis $SiO_2/H_2O$ von 1 : 5 bis 1 : 100 eingestellt und die Reaktionsmischung so lange bei einer Reaktionstemperatur von 70 bis 250°C gehalten, bis das Alkalischichtsilikat auskristallisiert ist. Man kann auf diese Weise beispielsweise ein Alkalischichtsilikat mit einem Molverhältnis $Na_2O/SiO_2$ von etwa 1 : 21 mit Kenyait-Struktur erhalten. Durch sauren Ionenaustausch ist daraus die entsprechende freie Säure herstellbar. Die so erhaltenen Verbindungen werden im folgenden als Na-SKS-1 bzw. H-SKS-1 bezeichnet.

In Gegenwart von Impfkristallen mit Magadiit-Struktur läßt sich nach diesem Verfahren auch ein Natriumschichtsilikat mit dem Molverhältnis $Na_2O/SiO_2$ von etwa 1 : 14 herstellen, das Magadiit-Struktur aufweist. Durch sauren Ionenaustausch erhält man hieraus die entsprechende freie Kieselsäure. Diese Verbindungen werden im folgenden als Na-SKS-2 und H-SKS-2 bezeichnet.

Es sind mehrere Schichtsilikate mit der ungefähren Zusammensetzung $Na_2Si_2O_5$ bekannt. Hierzu gehören die folgenden als Na-SKS-5, Na-SKS-6, Na-SKS-7 und Na-SKS-11 bezeichneten Produkte. Na-SKS-5 läßt sich herstellen gemäß Glastechnischen Ber. 37, 194 - 200 (1964). Das Produkt ähnelt im Röntgenbeugungsdiagramm $\alpha$-$Na_2Si_2O_5$. Das Röntgenspektrum hat gemäß der Aufstellung in Powder Diffraction File, Inorganic Phases, (Int. Centre f. Diffraction Data) Swarthmore 1983 die Nummer 22-1397.

Na-SKS-6 läßt sich herstellen gemäß Zeitschrift für Kristallogr. *129*, 396 - 404 (1969). Es ähnelt $\delta$-$Na_2Si_2O_5$.

Na-SKS-7 läßt sich herstellen gemäß Glastechn. Ber. *37*, 194- 200 (1964). Es ähnelt $\beta$-$Na_2Si_2O_5$.

Na-SKS-11 läßt sich herstellen gemäß Glastechn. Ser. *37*, 194 - 200 (1964), sowie gemäß Zeitschrift für Kristallogr. *129*, 396 - 404 (1969). Es ähnelt $\gamma$-$Na_2Si_2O_5$.

Ein technisch gangbarer Weg zur Herstellung von Na-SKS-5, Na-SKS-6 und Na-SKS-11 wird beschrieben in der deutschen Patentanmeldung P 3 417 649.7. Schichtsilikate mit anderer Zusammensetzung sind Na-SKS-9, Na-SKS-10 und Na-SKS-13.

Na-SKS-9 läßt sich herstellen gemäß Bull. Soc. franc. Min. Crist., *95*, 371 - 382 (1972). Es weist die ungefähre Zusammensetzung $NaHSi_2O_5 \cdot H_2O$ auf. Das Röntgenspektrum hat die Nummer 27-709.

Na-SKS-10 läßt sich herstellen gemäß Bull. Soc. franc. Min. Crist., *95*, 371 - 382 (1972) sowie gemäß Amer. Mineral., *62*, 763 - 771 (1977). Das Röntgenspektrum hat die Nummer 25-1309. Das Produkt hat die ungefähre Zusammensetzung $NaHSi_2O_5 \cdot 2H_2O$. Es ähnelt dem Mineral Kanemit für den in der Literatur die Formel $NaHSi_2O_4(OH)_2 \cdot 2H_2O$ angegeben ist, die $NaHSi_2O_5 \cdot 3H_2O$ entspricht.

Na-SKS-13 läßt sich herstellen gemäß Bull. Soc. franc. Min. Crist., *95*, 371 - 382 (1972). Das Röntgenspektrum hat die Nummer 27-708. Das Produkt hat die ungefähre Zusammensetzung $NaHSi_2O_5$.

Aufgabe der vorliegenden Erfindung ist es, Formkörper aus silikatischem Material bereitzustellen, die porös sind, leicht zu produzieren sind und eine gewisse mechanische Stabilität aufweisen, damit sie z. B. als Füllkörper in einer Kolonne zur Adsorption von Dämpfen aus Gasen verwendet werden können ohne zerdrückt zu werden.

Formkörper aus silikatischem Material werden üblicherweise unter Zusatz von Bindemitteln hergestellt. Damit wird der bei der Formgebung entstehende Formling soweit stabil, daß er wenigstens aus der Form entfernt und (schonend) transportiert werden kann, ohne zu zerfallen. Beim Verpressen von Quarzmehl wird Kalk als Bindemittel zugesetzt.

Es wurden nun Formkörper aus silikatischem Material gefunden, die dadurch gekennzeichnet sind, daß sie aus einer Kieselsäure mit Schichtstruktur oder deren Salzen mit der allgemeinen Formel $(H,M)_2Si_yO_{2y+1}$ oder den entsprechenden Hydraten bestehen, wobei M für Lithium, Natrium, Kalium oder Ammonium steht und y 1,7 bis 24 ist. Die Protonen der freien Kieselsäure können also ganz oder teilweise durch M ersetzt sein.

Die entsprechenden Hydrate haben die allgemeine Formel $(H,M)_2Si_yO_{2y+1} \cdot xH_2O$, wobei x eine Zahl zwischen 0 und 20 ist. In getrockneten Pulvern oder Formkörpern liegt x unter 7 und meist unter 4,5.

Auf die Unterscheidung zwischen verschiedenen Formen gebundenen Wassers wird hier bewußt verzichtet. Die allgemeine Formel $(H,M)_2Si_yO_{2y+1} \cdot xH_2O$ soll auch alle jene Schichtsilikate einschließen, die zwar in der Literatur evtl. mit OH-Gruppen formuliert worden sind, sich jedoch rechnerisch durch die gleiche allgemeine Formel beschreiben lassen. Der Verzicht auf eine gesonderte Kennzeichnung von Silanol-OH-Gruppen soll keineswegs bedeuten, daß solche in den Ausgangsverbindungen oder den erfindungsgemäßen Formkörpern nicht vorhanden wären.

Die Formkörper können auch aus einem Gemisch verschiedener Schichtkieselsäuren oder deren Salzen bestehen. Die erfindungsgemäßen Formkörper können unterschiedliche geometrische Form und Größe besitzen. Sie können z. B. die Form von Kugeln, Zylindern, Prismen, Würfeln, Quadern, Rohren oder Platten aufweisen.

Wegen der guten mechanischen Eigenschaften, insbesondere der Wasserbeständigkeit, sind Formkörper bevorzugt, deren silikatisches Material (Salz einer Schichtkieselsäure oder freie Schichtkieselsäure) Magadiit-Struktur oder Kenyait-Struktur aufweist. In diesem Fall sind auch die Ausgangsverbindungen leicht zugänglich.

Die erfindungsgemäßen Formkörper lassen sich dadurch herstellen, daß man die Schichtkieselsäuren oder deren Salze in Pulverform, gegebenenfalls nach Anfeuchten mit Wasser oder einem organischen Lösungsmittel, zu walzenförmigen Formlingen extrudiert und anschließend trocknet. Man kann auch die erzeugten walzenförmigen Formlinge auf einem Pelletierteller abrunden und erhält dann nach Trocknung bei 120°C formstabile, etwa kugelförmige Granalien. Diese haben eine Härte, die für viele Zwecke ausreichend ist. Bei der Prüfung mit dem Pfizer-Hardness-Tester werden Werte von 3 bis 115 kg beobachtet, die einem Prüfdruck von 10 bis 37 bar entsprechen.

Die erfindungsgemäßen Formkörper lassen sich auch dadurch herstellen, daß man die Schichtkieselsäuren oder deren Salze als trockene (oder mit Wasser oder organischen Lösemitteln) angefeuchtete Pulver in eine Form gibt und unter Anwendung von Druck zu Formkörpern verpreßt. Der entstandene Formkörper wird anschließend aus der Form entfernt. Die angewandten Drucke liegen höher als im Fall der oben erwähnten Extruder. Bei Einsatz angefeuchteter Pulver müssen die erzeugten Formkörper noch getrocknet werden. Es ist überraschend, daß sich nach diesem Verfahren ohne jeglichen Zusatz von Bindemitteln formstabile Körper herstellen lassen und diese eine noch höhere Härte aufweisen als die oben erwähnten Granalien. So läßt sich ein trockenes Pulver aus Schichtkieselsäuren oder Salzen von Schichtkieselsäuren in einem Rohr unter Belastung durch einen Stempel mit einem Preßdruck von z. B. 500 bar zu zylinderförmigen Körpern mit dem Durchmesser 16 mm verpressen. Bei diesen größeren Formkörpern eignet sich allerdings der Pfizer-Hardness-Tester nicht als Prüfwerkzeug. Stattdessen wurde in diesen Fällen die Shore-Härte gemessen, die bei den trockenen Formlingen, selbst ohne thermische Nachbehandlung, ohne weiteres Werte über 40 erreichen kann. Formkörper mit Shore-Härten von mindestens 40 sind wegen ihrer mechanischen Stabilität bevorzugt.

Es hat sich weiter gezeigt, daß durch ein mehrstündiges Erhitzen der Formkörper auf Temperaturen von 250 bis 1200°, vorzugsweise 300 bis 650°C, in vielen Fällen die Formkörper gegen Wasser oder wäßrige Lösungen beständig werden. Dies ist nützlich für die Verwendung der Formkörper als Katalysatorträger, wenn die zu katalysierende Umsetzung in Gegenwart von oder unter Bildung von Wasser stattfindet. Die Mindesttemperatur, die nötig ist, um diesen Effekt zu erreichen, hängt vom Material ab. Am besten geeignet sind hierfür die freien Schichtkieselsäuren. Formkörper aus H-SKS-2 sind schon nach einer dreistündigen Temperung bei 250°C wasserbeständig, während man bei Verwendung von H-SKS-1 und gleicher Zeitdauer 400°C benötigt. Formkörper aus Na-SKS-1 und Na-SKS-2 werden erst nach Temperung bei 600°C wasserbeständig. Die Lithium- und Kaliumsilikate verhalten sich ähnlich wie die Natriumsilikate. Die besonders alkalireichen Schichtsilikate Na-SKS-6, Na-SKS-10, Na-SKS-9 und Na-SKS-13 sind weniger geeignet zur Herstellung wasserfester Formkörper.

Die aufgenommenen Röntgenspektren beweisen, daß in allen Fällen trotz des veränderten Verhaltens gegenüber Wasser die getemperten Formkörper noch aus Schichtkieselsäuren bzw. deren Salzen bestehen. Die Dauer des Temperns ist nicht kritisch. In den meisten Fällen reicht ein Tempern von einer Stunde bereits aus. Ein Verlängern des Temperns über Stunden bringt keine weiteren Vorteile. Gleichzeitig wird durch das Tempern auch die Härte des Formkörpers weiter erhöht.

Im Hinblick auf die Härte der Formkörper ist ihre hohe Porosität überraschend. Die durch Verpressen und anschließendes Tempern hergestellten wasserfesten Körper weisen Porositäten von mindestens 40 %, gemessen nach DIN 51 056 auf, selbst wenn ohne jeglichen Zusatz von porenbildenden Stoffen gearbeitet wurde. In vielen Fällen lassen sich Porositäten von 50 bis 60 % feststellen, wenn man die Körper nach DIN 51 056 im Vakuum mit Wasser tränkt. Dies zeigt, daß es sich um offene Poren handelt und macht derartige Formkörper besonders geeignet zur Tränkung mit wäßrigen Lösungen oder Lösungen in organischen Lösemitteln. Die erfindungsgemäßen Formkörper sind deswegen geeignet als Träger für Katalysatoren. Beispielsweise kann man sie mit Metallsalzlösungen, wie z. B. Lösungen von Kupfernitrat, Nickelnitrat oder Kobaltacetat, tränken, anschließend durch Erhitzen das Lösungsmittel, insbesondere Wasser, verdunsten und die Metallsalze gegegebenenfalls zu ihren Oxiden zersetzen. Um die Tränkung der Formkörper zu fördern, können die Metallsalzlösungen nichtionische oder anionische Netzmittel, wie z. B. oxäthylierte Alkylphenole oder Alkylarylsulfonate enthalten. Ausgehend von Kupfernitrat erhält man auf diese Weise Hydrierungskatalysatoren, insbesondere für die Gasphase. Je höher die Porosität, desto kleiner ist die scheinbare Dichte. Bei Arbeitsdrucken von 500 bar erhält man Dichten von etwa 1,0. Mit Preßdrucken von 1000 - 2000 bar bei der Formgebung erhält man Formkörper höherer Dichte (1.2 - 1.3) und geringerer Porosität. Dichten von über 1.3 lassen sich erzeugen, wenn man die mit Pulver gefüllte Form vor und während des Preßvorganges evakuiert.

Überraschenderweise eignen sich die erfindungsgemäßen Formkörper auch ohne Behandlung mit Metallsalzlösungen als Katalysatoren für verschiedene Zwecke. Es wurde gefunden, daß sich die erfindungsgmäßen Formkörper, insbesondere Formkörper aus freien Schichtkieselsäuren, vorzugsweise Formkörper aus H-SKS-1, zur Dehydratisierung von hydroxylgruppenhaltigen aliphatischen Kohlenwasserstoffen in der Gasphase eignen. Dehydratisiert werden vorzugsweise einwertige aliphatische Alkohole mit einer Kettenlänge von $C_1$-$C_5$, insbesondere $C_1$-$C_2$ sowie Propanol, bei Temperaturen von 180° - 600°C, vorzugsweise 250 - 500°C. Der Alkoholdampf strömt dabei im allgemeinen mit einer Geschwindigkeit von 0,1 - 10 Raumteilen pro Raumteil (Schüttvolumen) Katalysator und Stunde durch das Katalysatorbett. Läßt man z. B. Methanoldampf bei 250 bis 450°C durch ein Rohr mit Granalien aus H-SKS-1 strömen, so erhält man als Umsetzungsprodukt hauptsächlich $C_2$- bis $C_4$-Alkene neben Dimethylether. Die bisher für diesen Zweck eingesetzten Katalysatoren auf Silikatbasis (Zeolithe) enthielten Aluminium und unterscheiden sich von den erfindungsgemäß eingesetzten Produkten weiterhin durch ihre dreidimensionale Gerüststruktur.

Die erfindungsgemäßen Formkörper eignen sich auch als Crack-Katalysatoren. Läßt man z. B. gesättigte aliphatische Kohlenwasserstoffe mit 3 bis 10 Kohlenstoffatomen bei Temperaturen von 400 bis 700°C über die erfindungsgemäßen Formkörper, insbesondere über Granalien aus freien Schichtkieselsäuren, strömen, so entstehen Alkene mit geringerer Kohlenstoff-Zahl sowie Methan.

Die erfindungsgemäßen Formkörper eignen sich weiterhin als Trockenmittel und/oder Absorptionsmittel für Dämpfe organischer Verbindungen.

Insbesondere eignen sich aus den Schichtsilikaten Na-SKS-1 und Na-SKS-2 hergestellte Granalien für die Adsorption von Wasser und damit zum Trocknen von Gasen ebenso wie für die Adsorption von Dämpfen organischer Substanzen, z. B. n-Hexan und Cyclohexan. Die Adsorption erfolgt im allgemeinen bei Temperaturen von 5 bis 95°C, vorzugsweise bei Raumtemperatur. Das Aufnahmevermögen der Granalien für die verschiedenen Dämpfe wird durch die vorherige Temperung der Granalien beeinflußt. So erhält man besonders hohe Werte für die Adsorption von Wasser (28 %) und Cyclohexan (32 %), wenn man aus Na-SKS-2 hergestellte Granalien vor ihrer Verwendung 4 Stunden bei 300°C tempert. Tempert man dieses Material dagegen bei 450°C, so nimmt das Adsorptionsvermögen wieder ab. Adsorbierend auf Dämpfe wirken außer den Formkörpern aus Schichtsilikaten auch solche aus den freien Schichtkieselsäuren, so z. B. aus H-SKS-6. Stellt man daraus Granalien her und tempert diese bei 300°C, so erzielt man eine gute Adsorption von Dämpfen des n-Hexans und des Cyclohexans sowie von Wasserdampf.

Aus pulverförmigen Schichtsilikaten wie z. B. Na-SKS-1 aber auch aus den entsprechenden Kieselsäuren wie z. B. H-SKS-1 lassen sich durch Pressen zwischen zwei Platten plattenförmige Körper herstellen. So erhält man bei einem Preßdruck von 600 bar aus Na-SKS-1 Platten, die nach Tempern bei 600°C eine Porosität von 55 %, eine Dichte von 1,05 und eine Shore-Härte von 55 aufweisen. Diese Platten eignen sich bis zu Temperaturen von 600°C gut als thermisches Isoliermaterial. Die gute isolierende Wirkung beschränkt sich nicht auf die Platten, sie ist vielmehr eine Eigenschaft aller erfindungsgemäßen Formkörper, wobei eine hohe Porosität (>50 %) besonders günstig ist.

Wie erwähnt, weisen die erfindungsgemäßen Formkörper eine hohe Porosität auf. Bei der Herstellung der Formkörper sind porenbildende Zusätze nur dann erforderlich, wenn besonders hohe Porositäten gewünscht werden; sie können jedoch auch für Formkörper mit niedrigen Porositäten eingesetzt werden. Die Porenbildner werden thermisch aus den Formkörpern entfernt. Als geeigneter porenbildender Zusatz hat sich Holzmehl erwiesen. Es verbrennt beim Tempern der Formkörper in oxidierender Atmosphäre (Luft) praktisch rückstandsfrei. Geeignet als Porenbildner sind auch Ammoniumcarbonat und Ammoniumbicarbonat. Beide Salze lassen sich in Mengen von 0 bis 25 %, z. B. 10 %, den pulverförmigen Schichtkieselsäuren (oder deren Salzen) vor der Formgebung zumischen. Sie verdampfen beim späteren Tempern.

Die Erfindung wird durch die Beispiele näher erläutert.

**Beispiel 1**

300 g Na-SKS-1 werden mit 240 g $H_2O$ angefeuchtet und durch einen Extruder gegeben. Es entstehen kurze Stränge von 3 mm Durchmesser und 4 mm Länge. Diese werden anschließend in einer Pelletiertrommel abgerundet zu Granalien ohne Kanten und bei 120°C 2 Std. getrocknet.

Die Härte, gemessen mit dem Hardness-Tester von Chas. Pfizer & Co., Inc., New York, beträgt 3 kg. Der Hardness-Tester ist eine Prüfzange für Tabletten. Unter Berücksichtigung der Stempelfläche von 3,1 mm², über die der Druck auf den Prüfkörper ausgeübt wird, ergibt sich für die Umrechnung der Meßwerte in bar ein Faktor von 3,2, d. h. die 3 kg entsprechen einem standgehaltenen Zangendruck von 9,6 bar.

**Beispiel 2**

400 g Na-SKS-2 werden mit 320 g $H_2O$ angefeuchtet und daraus Granalien hergestellt, wie in Beispiel 1 beschrieben. Die getrockneten Granalien halten im Hardness-Tester einen Druck von 10,5 bar aus.

**Beispiel 3**

200 g Na-SKS-6 werden mit 150 g $H_2O$ angefeuchtet und daraus Granalien hergestellt, wie in Beispiel 1 beschrieben. Die getrockneten Granalien halten im Hardness-Tester einen Druck von 37 bar aus.

**Beispiel 4**

R-SKS-1 wird gemäß Beispiel 28 durch Reaktion von Na-SKS-1 mit Salzsäure hergestellt. Aus dem gewaschenen, filterfeuchten Produkt werden Granalien hergestellt, wie in Beispiel 1 beschrieben. Die getrockneten Granalien halten im Hardness-Tester einen Druck von 20 bar aus.

**Beispiel 5**

H-SKS-2 wird gemäß Beispiel 30 durch Reaktion von Na-SKS-2 mit Salzsäure hergestellt. Aus dem gewaschenen, filterfeuchten Produkt werden Granalien hergestellt, wie in Beispiel 1 beschrieben. Die getrockneten Granalien halten im Hardness-Tester einen Druck von 17,6 bar aus.

**Beispiel 6**

Die im Beispiel 1 hergestellten Granalien aus Na-SKS-1 werden 3 Stunden bei 400°C getempert. Bei einer anschließenden mehrtägigen Lagerung in Wasser zerfallen sie nicht.

**Beispiel 7**

Die in Beispiel 2 hergestellten Granalien aus Na-SKS-2 werden 4 Stunden bei 400°C getempert. Die getemperten Granalien halten im Hardness-Tester einen Druck von 17,9 bar aus, sind also durch das Tempern härter geworden. Bei einer mehrtägigen Lagerung in Wasser zerfallen sie nicht.

**Beispiel 8**

(Vergleichsbeispiel) Es wurden 200 g gefällte Kieselsäure (Lieferant: Merck, 20 Darmstadt) mit 320 g $H_2O$ angefeuchtet. Es wurde versucht, daraus wie im Beispiel 1 beschrieben, Granalien herzustellen. Die erhaltenen Granalien zerfielen jedoch in feuchtem wie in trockenem Zustand so leicht, daß eine Prüfung der Härte nicht möglich war. Die mechanische Stabilität wird auch durch Tempern bei 400°C nicht verbessert.

**Beispiel 9**

5,01 g pulverförmiges, trockenes Na-SKS-1 werden in einer röhrenförmigen Presse mit 16 mm Innendurchmesser unter einem Stempeldruck von 500 bar 5 min verpreßt. Der so hergestellte 23,3 mm hohe, zylindrische Formkörper (Durchmesser 16,3 mm) hat die Shore-Härte (D nach DIN 53 505) 52 und die Dichte 1,03.

**Beispiel 10**

4,99 g Na-SKS-2 werden wie in Beispiel 9 zu einem zylindrischen Formkörper von 16,3 mm Durchmesser und 23,1 mm Höhe verpreßt. Der Formkörper hat die Shore-Härte 60 und die Dichte 1,04.

**Beispiel 11**

H-SKS-1 wird gemäß Beispiel 28 durch Reaktion von Na-SKS-1 mit Salzsäure hergestellt. Das gewaschene Produkt wird anschließend bei 100°C getrocknet. Davon werden 4,88 g wie in Beispiel 9 zu einem zylindrischen Formkörper von 16,3 mm Durchmesser und 22,5 mm Höhe verpreßt. Der Formkörper hat die Shore-Härte 45 und die Dichte 1,04.

**Beispiel 12**

H-SKS-2 wird gemäß Beispiel 30 durch Reaktion von Na-SKS-2 mit Salzsäure hergestellt. Das gewaschene Produkt wird anschließend bei 100°C getrocknet. Davon werden 4,94 g wie in Beispiel 9 zu einem zylindrischen Formkörper von 16,3 mm Durchmesser und 22,8 mm Höhe verpreßt. Der Formkörper hat die Shore-Härte 57 und die Dichte 1,04.

**Beispiel 13**

Der in Beispiel 9 hergestellte Formkörper aus Na-SKS-1 wird 4 Stunden bei 600°C getempert. Er hat dann die Shore-Härte 55 und die Dichte 0,98. Der Körper ist nunmehr wasserbeständig. Seine offene Porosität, geprüft nach DIN 51 056-A-2 (Tränkung unter Vakuum), beträgt 0,56 cm$^3$/cm$^3$ bzw. 56 %.

**Beispiel 14**

Ein wie in Beispiel 10 hergestellter Formkörper aus Na-SKS-2 wird 4 Stunden bei 600°C getempert. Er hat dann die Shore-Härte 75 und die Dichte 1,07. Der Körper ist nunmehr wasserbeständig. Seine offene Porosität (geprüft wie in Beispiel 13) beträgt 58 %.

**Beispiel 15**

Ein wie in Beispiel 11 hergestellter Formkörper aus H-SKS-1 wird 4 Stunden bei 400°C getempert. Er hat dann die Shore-Härte 55, die Dichte 1,02 und ist wasserbeständig. Die offene Porosität (geprüft wie in Beispiel 13) beträgt 56 %. Das Röntgenspektrum zeigt, daß die Struktur des H-SKS-1 erhalten geblieben ist.

**Beispiel 16**

Ein wie in Beispiel 12 hergestellter Formkörper aus H-SKS-2 wird 4 Stunden bei 250°C getempert. Er hat dann die Shore-Härte 48 und die Dichte 1,03. Seine offene Porosität (geprüft wie in Beispiel 13) beträgt 60 %. Ein weiterer Formkörper aus H-SKS-2 wird statt dessen 4 Stunden bei 400°C getempert. Die Shore-Härte beträgt dann 60, die Dichte 1,0 und die offene Porosität 56 %.

In beiden Fällen bleibt die Struktur des H-SKS-2 erhalten, wie die Röntgenspektren beweisen. Beide Formkörper sind wasserbeständig.

**Beispiel 17**

Aus H-SKS-1 werden durch Pressen in einem zylinderförmigen Rohr mit Stempel Formkörper hergestellt, die einen Durchmesser von 51,4 mm und eine Höhe von ca. 49 mm haben. Mit erhöhtem Preßdruck des Stempels erhöht sich auch die Dichte der Formkörper:

Druck 480 bar  : Dichte 1,05
Druck 960 bar  : Dichte 1,21
Druck 1440 bar : Dichte 1,29

Wird vor und während des Preßvorganges Vakuum angelegt, läßt sich die Dichte noch weiter erhöhen. Sie beträgt dann nach Pressen mit 960 bar 1,36.

**Beispiel 18**

5,05 g Na-SKS-6 werden wie in Beispiel 9 zu einem zylindrischen Formkörper von 16,2 mm Durchmesser und 21,9 mm Höhe verpreßt. Die Shore-Härte beträgt 45, die Dichte 1,12.

**Beispiel 19** (Vergleichsbeispiel) Es wurden 5 g pulverförmige gefällte Kieselsäure (FK 320 DS von Degussa) wie in Beispiel 9 verpreßt. Der Formkörper zerfällt beim Entfernen aus der Form.

**Beispiel 20**

Pulverförmiges Na-SKS-1 wird in dünner Schicht zwischen 2 Platten trocken verpreßt. Der Preßdruck beträgt 600 bar. Es entsteht eine runde Platte von 3,1 mm Dicke und ca. 70 mm Durchmesser. Die Dichte liegt bei 1,1. Nach Tempern bei 600°C beträgt die Dichte 1,05 und die 55 %. Die Platte ist als thermisches Isoliermaterial zumindest bis 600°C geeignet.

**Beispiel 21**

Ein in Beispiel 13 beschriebener, bei 600°C getemperter Formkörper aus Na-SKS-1 wird mit 0,6 cm$^3$/cm$^3$ einer 10-%-igen Lösung von Cu (NO$_3$)$_2$·3H$_2$O im Vakuum getränkt. Der getränkte Formkörper wird getrocknet und anschließend 2 Stunden bei 350°C gehalten. Das Kupfernitrat zersetzt sich dabei zum CuO. Nach Reduktion ins Wasserstoff/Stickstoff-Gemisch ist der Formkörper als Hydrierungskatalysator für die Gasphase geeignet.

Hydrierungskatalysatoren ähnlicher Wirksamkeit lassen sich erhalten, wenn man pulverförmige Schichtsilikate oder Schichtkieselsäuren mit Lösungen von Metallsalzen, z. B. von Kupfernitrat, tränkt, trocknet und anschließend unter Druck zu Formkörpern verarbeitet. Sowohl bei der Tränkung von Formkörpern als auch von Pulvern aus Schichtsilikaten kann ein Teil der Alkaliionen durch Cu$^{++}$-ionen ausgetauscht werden. Dieser Effekt läßt sich über den pH-Wert der Tränklösung steuern.

**Beispiel 22**

Die im Beispiel 1 beschriebenen Granalien aus Na-SKS-1 eignen sich zur Adsorption von Dämpfen aus der Gasphase. Geprüft wurde das Adsorptionsvermögen, indem die Granalien jeweils 15 Stunden im Exsikkator über Schalen mit Hexan oder Cyclohexan oder 20-%-iger Schwefelsäure (H$_2$O-Partialdruck ca. 20 mbar) aufbewahrt wurden. Anschließend wurde die Gewichtszunahme der Granalien festgestellt. Die Ergebnisse finden sich in der nachfolgenden Tabelle. Auch Granalien aus Na-SKS-1, die bei 400°C oder 600°C getempert wurden, adsorbieren gut. Ebenfalls geeignet sind Granalien aus Na-SKS-2, die bei 300°C oder 450°C getempert wurden. Ein überraschend gutes Adsorptionsvermögen für Dämpfe zeigen schließlich Granalien aus H-SKS-6.

Das Ausgangsmaterial für Granalien aus H-SKS-6 wird wie folgt erzeugt: Aus Na-SKS-6 wird durch Reaktion mit Salzsäure die freie Kieselsäure (H-SKS-6) hergestellt.

Das gewaschene Produkt wird anschließend bei 120° getrocknet. Es hat das Röntgenspektrum mit der Nummer 27-606. Als Formel wird H$_2$Si$_2$O$_5$ angegeben (Johan und Maglione, Bull. Soc. franc. Min. Crist. *95*, 371 - 82 (1972)). Die Granalien werden aus dem angefeuchteten H-SKS-6 nach der Methode von Beispiel 1 hergestellt und anschließend bei 300°C getempert.

| Material | Temperung °C | Adsorption (Gew.-%) | | |
| --- | --- | --- | --- | --- |
| | | Hexan | Cyclohexan | Wasser |
| Na-SKS-1 | 120 (Trocknen) | 11 | 12,3 | 10,6 |
| Na-SKS-1 | 400 | 11,3 | 11,5 | 16,6 |
| Na-SKS-1 | 600 | 10,3 | 11,1 | 16,5 |
| Na-SKS-2 | 300 | 8,9 | 32,4 | 28,1 |
| Na-SKS-2 | 450 | 17,1 | 13,8 | 16,0 |
| H-SKS-6 | 300 | 16,5 | 33,1 | 21,1 |

**Beispiel 23**

Aus H-SKS-1 werden Granalien hergestellt wie im Beispiel 1 angegeben. In ein beheizbares senkrecht stehendes Rohr wird eine Schüttung von 100 ml dieser Granalien eingefüllt. Die Temperatur der Schüttung wird auf 400 - 450°C gehalten. Von oben wird Methanol-Dampf über die Schüttung geführt. Das Reaktionsgemisch wird nach Verlassen des Rohres gekühlt, um entstandenes Wasser auszukondensieren. Das verbleibende Gas wird im Gaschromatographen untersucht. Es enthält im wesentlichen Ethen, Propen und die verschiedenen Butene sowie Dimethylether. Auch sind geringe Mengen an Aromaten nachweisbar.

**Beispiel 24**

Aus H-SKS-2 werden Granalien hergestellt wie in Beispiel 1 beschrieben. In ein senkrecht beheizbares Rohr werden 100 ml einer Schüttung der Granalien eingefüllt. Die Schüttung wird auf 518°C aufgeheizt und während der Umsetzung bei dieser Temperatur gehalten. n-Hexan-Dampf wird von oben über die Schüttung geführt. Das Reaktionsgemisch wird nach Verlassen des Rohres abgekühlt, um die Hauptmenge an n-Hexan auszukondensieren. Das verbleibende Gas wird im Gaschromatographen untersucht. Es hat folgende Zusammensetzung:

| | |
| --- | --- |
| H$_2$ | 0,4 % |
| CH$_4$ | 10 % |
| C$_2$H$_6$ | 7 % |
| C$_2$H$_4$ | 18,5 % |
| C$_3$H$_8$ | 1,4 % |
| C$_3$H$_6$ | 16,4 % |
| i-C$_4$H$_{10}$ | 0,05 % |

| n-C$_4$H$_{10}$ | 0,3 % |
|---|---|
| ΣC$_4$H$_8$ | 8,5 % |
| ΣC$_5$ | 2,5 % |
| n-C$_6$H$_{14}$ | 30 % |
| ΣC$_6$ | 1,8 % |
| ΣC$_7$ | 0,1 % |

**Beispiel 25**

200 g H-SKS-1 werden mit 140 ml Ethanol angefeuchtet und daraus Granalien hergestellt, wie in Beispiel 1 beschrieben. Die bei 120°C getrockneten Granalien halten im Hardness-Tester einen Druck von 13 bar aus.

**Beispiel 26**

200 g H-SKS-1 werden mit 167 g Kieselsol (mit 30 % SiO$_2$) vermischt und aus dieser Mischung Granalien hergestellt, wie in Beispiel 1 beschrieben. Die bei 120°C getrockneten Granalien halten im Hardness-Tester einen Druck von 30 bar aus.

**Beispiel 27**

(Darstellung von Na-SKS-1)
Man stellt zunächst eine Reaktionsmischung der molaren Zusammensetzung

0,303 Na$_2$O : 0,0052 Al$_2$O$_3$ : SiO$_2$ : 30 H$_2$O

dadurch her, daß man 83,5 Gewichtsteile Natronwasserglas (27 % SiO$_2$, 8,43 % Na$_2$O, 0,24 % Al$_2$O$_3$) zu 149 Teilen Wasser gibt. Danach wird ein Teil eines filterfeuchten kristallinen Natriumsilikats aus einem früheren Versuch (71 % Gewichtsverlust durch Erhitzen auf 1200°C; für die Berechnung der molaren Zusammensetzung wurde nur der Wasseranteil berücksichtigt) zugegeben. Man setzt anschließend langsam unter Rühren 4,93 Teile 96-%-ige Schwefelsäure zu. Danach hat die Reaktionsmischung folgende molare Zusammensetzung:

0,174 Na$_2$O : 0,0052 Al$_2$O$_3$ : SiO$_2$ : 0,129 Na$_2$SO$_4$ : 30 H$_2$O.

Die Reaktionsmischung wird in einem Edelstahl-Autoklaven innerhalb von 1,5 Stunden auf 205°C erhitzt. 2,5 Stunden bei dieser Temperatur gehalten und anschließend langsam abgekühlt. Nach dem Abkühlen wird die Reaktionsmischung filtriert, mit Wasser gewaschen und auf einer Nutsche trocken gesaugt. Das filterfeuchte Produkt weist einen Glühverlust von 55 % auf. Das an der Luft kurzzeitig getrocknete Produkt wird thermogravimetrisch untersucht. Bis zu einer Temperatur von etwa 140°C ist ein Gewichtsverlust von 43 % eingetreten. Bis ca. 1000°C wird keine weitere wesentliche Gewichtsabnahme beobachtet. Das bei 120°C bis zur Gewichtskonstanz getrocknete Produkt, Na-SKS-1, zeigt folgende elementar-analytische Zusammensetzung: 3,8 % Na, 0,24 % Al, 41,5 % Si und 0,003 % Fe. Es läßt sich daraus ein molares SiO$_2$/Na$_2$O-Verhältnis von 17,9 errechnen. Das molare SiO$_2$/Al$_2$O$_3$-Verhältnis von 332 zeigt, daß trotz der Anwesenheit von gelöstem Al$_2$O$_3$ in der Reaktionsmischung dieses nur in sehr geringen Mengen in das Endprodukt eingebaut wird.

**Beispiel 28**

(Darstellung von H-SKS-1)
Das kristalline Na-Silikat aus Beispiel 27 wird zweimal mit 5-%-iger Salzsäure bei 80°C 15 Minuten lang extrahiert. Man wäscht, filtriert und trocknet bei 40°C. Die Untersuchung der differentiellen Thermoanalyse ergibt eine ausgeprägte endotherme Umwandlung bei etwa 120°C und eine weit weniger ausgeprägte endotherme Umwandlung bei etwa 1180°C.

**Beispiel 29**

(Darstellung von Na-SKS-2)
Das Produkt wird mit gleicher Eduktzusammensetzung wie in Beispiel 27 hergestellt. Der Reaktionsmischung werden Impfkristalle eines Magadiit-artigen Silikats aus einem früheren Versuch zugesetzt. Die Reaktionsmischung wird 19 Stunden bei 165°C gerührt, nach dem Erkalten filtriert, mit Wasser gewaschen und auf einer Nutsche trockengesaugt. 10 g der Mutterlauge der Reaktionsmischung, mit 250 ml Wasser verdünnt, haben einen pH-Wert von 10,4. Das filterfeuchte Produkt, welches beim Glühen (>1000°C) 61,3 % seines Gewichtes verliert, wird mit Schwefelsäure titriert und aus dem Wendepunkt der Titrationskurve bei pH 5,0 ein Äquivalenzwert von 215 meq/100 g geglühtes Produkt ermittelt. Für ein Produkt der Zusammensetzung $Na_2O \cdot ySiO_2$ wird hieraus ein Ionenaustauschvermögen von 138 mmol $Na^+$/mol $SiO_2$, entsprechend einem $SiO_2$ : $Na_2O$-Verhältnis von 14,5 : 1 ermittelt. Arbeitet man ohne Impfkristalle, so werden deutlich längere Reaktionszeiten erforderlich.

**Beispiel 30**

(Darstellung von H-SKS-2)

100 g feuchtes Produkt aus Beispiel 29 werden zu 200 ml 5-%-iger Salzsäure gegeben und 1,25 Stunden bei Raumtemperatur gerührt. Das Produkt wird filtriert, erneut zu einer gleichen Menge Salzsäure gegeben, 25 Stunden gerührt, filtriert und zweimal gründlich mit Wasser gewaschen, wobei das Produkt mit Wasser gerührt wird und bei der Filtration gewaschen wird. Anschließend wird das Produkt trockengesaugt. Es weist einen Glühverlust von 57 % auf. Von dem trockengesaugten Produkt werden 10 g zu 1900 ml 5-%-iger NaCl-Lösung gegeben und anschließend mit 1m NaOH titriert. Bei der graphischen Darstellung der Titrationswerte wird aus dem Wendepunkt der Kurve bei pH 8,3 ein Äquivalenzwert von 235 mmol $H^+$/100 g geglühtes Produkt ermittelt. Daraus wird ein Ionenaustauschvermögen von etwa 144 meq/mol $SiO_2$, entsprechend einem $SiO_2$ : $Na_2O$-Verhältnis bzw. einem Verhältnis $SiO_2/2H^+$ von 13,9 : 1 bestimmt.

**Beispiel 31**

Die Granalien von Beispiel 2 wurden zur Entwässerung 4 Stunden bei 300° getempert und dann zum Trocknen von Lösemitteln eingesetzt. Hierzu wurden jeweils 5 g der Granalien zu 25 ml von mit Wasser gesättigtem Essigsäureäthylester (o-Xylol, n-Hexan) gegeben. Nach 3 Stunden war der Wassergehalt des Lösemittels von 3,35 (0,056 %, 0,017 %) auf 0,63 % (0,0067 %, 0,0045 %) gesunken.

**Beispiel 32**

Ein durch Verpressen von Na-SKS-2 bei 400 bar erzeugter Zylinder von 50 mm Durchmesser und 48 mm Höhe wurde auf eine Heizplatte gelegt. Thermoelemente wurden unmittelbar auf der Platte und auf der Oberseite des Zylinders befestigt. Die Platte wurde auf 162 ± 2°C aufgeheizt. Auf der Oberseite des Zylinders stellte sich eine Temperatur von 45°C ein, die 90 min konstant blieb. Danach wurde die Heizplatte abgeschaltet und der Zylinder entfernt ohne die Position der Heizelemente zu ändern. Innerhalb von 23 min sank die Temperatur auf der Platte um 2°C. Dagegen stieg in dieser Zeit die Temperatur am oberen Thermoelement kontinuierlich bis 55°C an.

**Patentansprüche**

1. Formkörper aus silikatischem Material, dadurch gekennzeichnet, daß sie aus einer Kieselsäure mit Schichtstruktur oder deren Salzen mit der allgemeinen Formel $(H,M)_2Si_yO_{2y+1}$ oder den entsprechenden Hydraten bestehen, wobei M für Lithium, Natrium, Kalium oder Ammonium steht und y 1,7 bis 24 ist.

2. Formkörper gemäß Anspruch 1, dadurch gekennzeichnet, daß sie eine Shore-Härte von über 40 aufweisen.

3. Formkörper gemäß Anspruch 1, dadurch gekennzeichnet, daß sie eine Porosität von mindestens 40 %, gemessen nach DIN 51 056-A-2 aufweisen.

4. Verfahren zur Herstellung von Formkörpern gemäß Anspruch 1, dadurch gekennzeichnet, daß man pulverförmige Schichtkieselsäuren oder deren Salze der allgemeinen Formel $(H,M)_2Si_yO_{2y+1}$ oder die entsprechenden Hydrate, wobei M für Lithium, Natrium, Kalium oder Ammonium steht, und y 1,7 bis 24 ist, gegebenenfalls nach Anfeuchten mit Wasser oder einem organischen Lösungsmittel zu walzenförmigen Formlingen extrudiert und anschließend trocknet.

5. Verfahren zur Herstellung von Formkörpern gemäß Anspruch 1, dadurch gekennzeichnet, daß man Schichtkieselsäuren oder deren Salze der allgemeinen Formel $(H,M)_2Si_yO_{2y+1}$ oder die entsprechenden Hydrate, wobei M für Lithium, Natrium, Kalium oder Ammonium steht, und y 1,7 bis 24 ist, als trockene oder feuchte Pulver in eine Form gibt, unter Anwendung von Druck zu Formkörpern verpreßt und gegebenenfalls trocknet.

6. Verfahren gemäß Anspruch 5, dadurch gekennzeichnet, daß man die Formkörper bei Temperaturen über 250°C tempert.

7. Verwendung der Formkörper gemäß Anspruch 1 als Träger für Katalysatoren.

8. Verwendung der Formkörper gemäß Anspruch 1 als Katalysator zur Dehydratisierung von hydroxylgruppenhaltigen aliphatischen Kohlenwasserstoffen in der Gasphase.

9. Verwendung der Formkörper gemäß Anspruch 1 als thermisches Isoliermaterial.

10. Verwendung der Formkörper gemäß Anspruch 1 als Trockenmittel und/oder Mittel zur Adsorption organischer Dämpfe.

11. Verwendung der Formkörper gemäß Anspruch 1 als Crack-Katalysatoren.

## Claims

1. A molding made of silicate material, which is composed of a silicic acid with a layer structure, or a salt thereof with the general formula $(H,M)_2Si_yO_{2y+1}$, or the corresponding hydrate, M representing lithium, sodium, potassium or ammonium and y being 1.7 to 24.

2. A molding as claimed in claim 1, which has a Shore hardness of more than 40.

3. A molding as claimed in claim 1, which has a porosity of at least 40 %, measured according to DIN 51 056-A-2.

4. A process for the preparation of moldings as claimed in claim 1, which comprises extruding pulverulent layer silicic acids, or salts thereof with the general formula $(H,M)_2Si_yO_{2y+1}$, or the corresponding hydrates, M representing lithium, sodium, potassium or ammonium and y being 1.7 to 24, to produce cylindrical blanks, if appropriate after moistening with water or an organic solvent, and then drying the blanks.

5. A process for the preparation of moldings as claimed in claim 1, which comprises introducing layer silicic acids, or salts thereof with the general formula $(H,M)_2Si_yO_{2y+1}$, or the corresponding hydrates, M representing lithium, sodium, potassium or ammonium and y being 1.7 to 24, into a mold, as dry or moist powders, converting them to moldings with the application of pressure and, if appropriate, drying the moldings.

6. The process as claimed in claim 5, wherein the moldings are heated at temperatures above 250°C.

7. The use of the moldings as claimed in claim 1 as catalyst supports.

8. The use of the moldings as claimed in claim 1 as catalysts for the dehydration, in the gas phase, of aliphatic hydrocarbons containing hydroxyl groups.

9. The use of the moldings as claimed in claim 1 as thermal insulating material.

10. The use of the moldings as claimed in claim 1 as drying agents and/or agents for the adsorption of organic vapors.

11. The use of the moldings as claimed in claim 1 as cracking catalysts.

## Revendications

1. Corps façonnés en un matériau de type silicate, caractérisés en ce qu'ils sont constitués d'un acide silicique à structure lamellaire ou de ses sels, de formule générale $(H,M)_2Si_yO_{2y+1}$, ou des hydrates correspondants, où M est le lithium, le sodium, le potassium ou l'ammonium, et y vaut de 1,7 à 24.

2. Corps façonnés selon la revendication 1, caractérisés en ce qu'ils ont une dureté Shore supérieure à 40.

3. Corps façonnés selon la revendication 1, caractérisés en ce qu'ils ont une porosité d'au moins 40 %, mesurée selon DIN 51 056-A-2.

4. Procédé pour la préparation de corps façonnés selon la revendication 1, caractérisé en ce qu'on extrude des acides siliciques lamellaires pulvérulents, ou leurs sels, de formule générale $(H,M)_2Si_yO_{2y+1}$, ou les hydrates correspondants, où M est le lithium, le sodium, le potassium ou l'ammonium, et y vaut de 1,7 à 24, éventuellement après humidification à l'eau ou à l'aide d'un solvant organique, pour donner des ébauches cylindriques, puis on sèche.

5. Procédé pour la préparation de corps façonnés selon la revendication 1, caractérisé en ce qu'on place dans un moule, sous forme de poudre sèche ou humide, des acides siliciques lamellaires ou leurs sels de formule générale $(H,M)_2Si_yO_{2y+1}$, ou les hydrates correspondants, où M est le lithium, le sodium, le potassium ou l'ammonium et y vaut de 1,7 à 24, on comprime en appliquant une pression pour donner des corps façonnés, et éventuellement on sèche.

6. Procédé selon la revendication 5, caractérisé en ce qu'on recuit les corps façonnés à des températures supérieures à 250°C.

7. Utilisation des corps façonnés selon la revendication 1, en tant que supports de catalyseurs.

8. Utilisation des corps façonnés selon la revendication 1, en tant que catalyseurs pour déshydrater en phase gazeuse des hydrocarbures aliphatiques contenant des groupes hydroxyle.

9. Utilisation des corps façonnés selon la revendication 1 en tant que matériaux isolants thermiques.

10. Utilisation des corps façonnés selon la revendication 1, en tant que dessiccants et/ou agents d'adsorption de vapeurs organiques.

11. Utilisation des corps façonnés selon la revendication 1 en tant que catalyseurs de craquage.